# FASCICULE DE BREVET EUROPEEN

(11) **EP 1 382 322 B1**
(45) Date de publication et mention de la délivrance du brevet: **12.09.2012**
(21) Numéro de dépôt: 03291701.5
(22) Date de dépôt: 09.07.2003
(51) Int. Cl.: A61K 8/02, A61K 8/11, A61K 8/19, A61Q 1/02, A61Q 3/02

(54) **Composition cosmétique de maquillage**
Kosmetische Schminkzusammensetzung
Make up cosmetic composition

(30) Priorité: 19.07.2002 FR 0209245
(43) Date de publication de la demande: 21.01.2004
(73) Titulaire: L'Oréal, 75008 Paris (FR)
(72) Inventeur: Simon, Jean-Christophe, 75012 Paris (FR); Girier-Dufournier, Franck, 75011 Paris (FR); Lemann, Patricia, 94000 Creteil (FR)
(74) Mandataire: Le Coupanec, Pascale A.M.P.

(56) Documents cités:
- EP-A- 0 792 637
- EP-A- 0 953 330
- EP-A- 1 195 155
- WO-A-00/49995
- WO-A-00/75240
- WO-A-01/51015
- WO-A-98/53011

## Description

La présente invention se rapporte au maquillage de la peau, par exemple celle du visage ou du corps, des lèvres ou des phanères tels que les cils, les sourcils, les ongles et les cheveux.

Les compositions de maquillage, telles que par exemple les poudres libres, les fonds de teint, les vernis à ongles, ls mascaras, les fards à joues, les fards à paupières, les rouges à lèvres, les gloss en pot ou les gloss liquides sont généralement constituées d'un milieu physiologiquement acceptable et de différents agents de coloration.

Depuis longtemps, les consommateurs recherchent des compositions qui permettent de remodeler le visage, en particulier de mettre en valeur les pommettes et/ou de rendre les lèvres pulpeuses. Il n'existe pas à ce jour de solution efficace pour répondre à cette attente.

Il est connu qu'un effet de volume peut être produit en appliquant une teinte claire et une teinte foncée côte à côte, la teinte claire étant appliquée sur la zone que l'on souhaite mettre en valeur. L'obtention de cet effet nécessite traditionnellement l'emploi de deux compositions différentes et dépend de l'habileté de celui qui les appliquent. Cette technique est de plus difficile à mettre en oeuvre pour le maquillage des lèvres.

Récemment, la faculté manifestée naturellement par les pigments goniochromatiques de changer de couleur selon l'angle d'observation et/ou d'incidence de la lumière a été mise à profit dans le domaine de la cosmétique. C'est ainsi que la demanderesse a proposé dans la demande EP-A-0 953 330 un kit de maquillage associant un premier pigment goniochromatique et un second pigment ayant l'une des couleurs du premier pigment. Cette association procure des effets colorés originaux sans toutefois modifier de manière importante la perception du volume de la partie du corps sur laquelle elle est appliquée.

Par ailleurs, la demande internationale WO 01/51015 propose des compositions qui associent à des pigments interférentiels conventionnels un pigment interférentiel à quatre couches, encore dénommé « shadow pigment », qui présente une coloration variable selon l'angle de réflexion spéculaire. Ceci se traduit au niveau du support traité par un changement de couleur entre le clair et le foncé. Ces compositions améliorent la perception du contour des différentes parties du visage ou du corps, mais elles ne créent pas un effet de volume satisfaisant.

Il existe par conséquent un besoin pour une composition cosmétique capable de procurer une impression de volume satisfaisante.

La présente invention vise à proposer des compositions permettant d'obtenir de nouveaux effets de maquillage, et notamment des compositions de maquillage qui créent un effet optique de volume une fois appliquées sur un support tel que la peau, les lèvres ou les phanères. Ces compositions, après application par exemple sur les joues, les paupières ou les lèvres, procurent une perception du volume différente de celle du support non maquillé. Un tel effet pourra être qualifié de « tridimensionnel » et plus particulièrement de "pulping" pour les lèvres ou de "morphing" pour le visage et le corps.

L'invention a ainsi pour objet, selon l'un de ses aspects, une composition cosmétique comportant, dans un milieu physiologiquement acceptable, au moins un agent de coloration goniochromatique en une quantité allant de 2 à 15 % par rapport au poids total de la composition et au moins des particules réfléchissantes distinctes de l'agent de coloration goniochromatique, ces dernières étant des particules de verre enrobé au moins partiellement par au moins une couche d'au moins un composé métallique, notamment d'un oxyde métallique.

Il a pu être constaté que l'association d'un agent de coloration goniochromatique et de particules réfléchissantes telles que définies ci-dessus permettait, de manière inattendue, de créer ou de renforcer l'impression de volume, les particules réfléchissantes générant des points de surbrillance visibles à l'oeil nu lorsque la composition est appliquée pour former une couche sur un support et éclairée.

Par « composition cosmétique », on désigne une composition telle que définie dans la Directive 93/35/CEE du Conseil du 14 juin 1993.

Par « milieu physiologiquement acceptable », on désigne un milieu non toxique et susceptible d'être appliqué sur la peau, les lèvres ou les phanères d'êtres humains.

Par « agent de coloration goniochromatique », on désigne au sens de la présente invention un agent de coloration permettant d'obtenir, lorsque la composition cosmétique est étalée sur un support, un trajet de couleur dans le plan a^{*}b^{*} de l'espace colorimétrique CIE 1976 qui correspond à une variation Dh de l'angle de teinte h d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la composition cosmétique ait été étalée à l'état fluide avec une épaisseur de 300 µm au moyen d'un étaleur automatique sur une carte de contraste de marque ERICHSEN et de référence Typ 24/5, la mesure étant effectuée sur le fond noir de la carte.

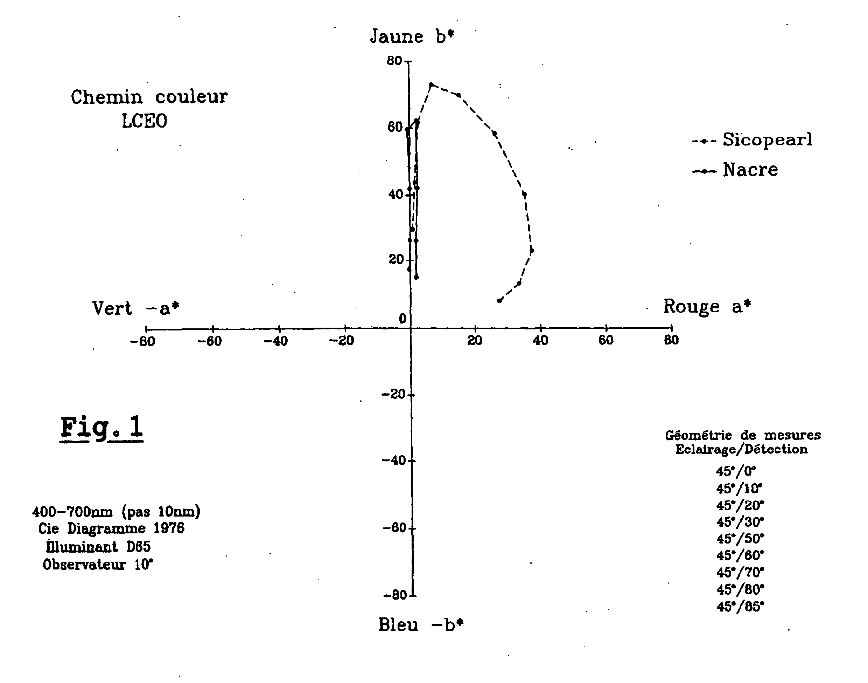

A titre d'exemple, on a représenté à la figure 1 un trajet de couleur obtenu avec un tel spectrogonioréflectomètre pour un gloss liquide réalisé conformément à l'invention, comportant des pigments goniochromatiques SICOPEARL® commercialisés par la société BASF.

Un agent de coloration goniochromatique au sens de la présente invention permet d'observer un changement de couleur, encore appelé « color flop », en fonction de l'angle d'observation, supérieur à celui que l'on peut rencontrer avec des nacres.

La figure 1 représente également pour comparaison le chemin de couleur pour une nacre SUMMIT GOLD YD30D commercialisée par la société ENGELHARD.

Par « particules réfléchissantes », on désigne au sens de la présente invention des particules dont la taille, la structure, notamment l'épaisseur de la ou des couches qui la constituent et leur natures physique et chimique, et l'état de surface, leur permettent de réfléchir la lumière incidente avec une intensité suffisante pour pouvoir créer à la surface de la composition revendiquée, lorsque cette dernière est appliquée sur le support à maquiller, des points de surbrillance visibles à l'oeil nu, c'est-à-dire des points plus lumineux qui contrastent avec leur environnement en semblant briller.

Les particules réfléchissantes peuvent troubler la perception visuelle de la courbure du support maquillé, en tendant à empêcher une focalisation visuelle durable, les points de surbrillance étant susceptibles d'apparaître ou de disparaître de manière aléatoire lorsque le support maquillé et l'observateur sont animés.

Comme cela sera précisé plus loin, il peut s'avérer souhaitable que la brillance moyenne de la composition dépasse un certain seuil, notamment lorsque la composition est destinée à être appliquée sur les lèvres. En effet, lorsque la composition présente une brillance relativement élevée, le support traité semble se détacher visuellement encore davantage de son environnement.

Par «brillance moyenne», on désigne la brillance telle qu'elle peut être mesurée à l'aide d'un brillancemètre, de manière conventionnelle, par la méthode suivante.

Sur une carte de contraste de marque LENETA et de référence FORM 1A PENOPAC, on étale une couche de 50 µm d'épaisseur de la composition dont on cherche à évaluer la brillance moyenne, à l'aide d'un étaleur automatique. La couche recouvre au moins le fond blanc de la carte. On procède de suite à la mesure de la brillance à 20° sur le fond blanc à l'aide d'un brillancemètre de marque BYK GARDNER et de référence microTRI-GLOSS.

La brillance moyenne de la composition est avantageusement supérieure ou égale à 30, voire 50 ou mieux 70, notamment lorsque la composition est destinée à être appliquée sur les lèvres.

La composition peut comporter une base brillante destinée à permettre d'obtenir la brillance moyenne souhaitée.

Par « base », on désigne au sens de la présente invention la composition cosmétique sans le ou les agents de coloration goniochromatique(s) et sans les particules réfléchissantes.

La composition cosmétique peut ainsi comporter, dans un exemple de mise en oeuvre, une base dont la brillance moyenne est supérieure à 20, voire à 50 ou même à 70 notamment dans le cas d'une composition destinée à être appliquée sur les lèvres. La présence du ou des agents de coloration goniochromatique(s) et des particules réfléchissantes dans la base brillante peut conduire à une composition cosmétique dont la brillance moyenne peut être différente ou non de la brillance moyenne de la base considérée isolément.

La formulation de la base dépendra de l'utilisation qui est faite de la composition cosmétique et de la forme sous laquelle la composition cosmétique est proposée.

La formulation de la base pourra ainsi être différente selon que la composition cosmétique est destinée à former un gloss liquide ou un rouge à lèvres, par exemple. On pourra par exemple choisir une base de rouge à lèvres de brillance moyenne égale à 60 environ, une base de gloss liquide ou de fard à paupières de brillance moyenne égale à 70 environ, et une base de vernis à ongles de brillance moyenne égale à 50 environ.

On désigne par « gloss liquide », encore appelé rouge à lèvres liquide ou brillant à lèvres, un produit fluide destiné à être appliqué sur les lèvres et conditionné par exemple dans un récipient pourvu d'un applicateur, cet applicateur comportant un organe de préhension qui sert également de capuchon de fermeture du récipient, et un élément d'application.

La composition cosmétique peut comporter, notamment dans le cas où elle est destinée à être appliquée sur les lèvres, une phase huileuse, notamment une phase huileuse ayant un indice de réfraction compris entre 1,47 et 1,51, ce qui peut permettre d'obtenir une brillance relativement élevée.

L'emploi d'une base brillante ne constitue qu'un moyen parmi d'autres d'obtenir une composition cosmétique présentant de la brillance. On ne sort pas du cadre de la présente invention lorsqu'avant ou après avoir appliqué une composition cosmétique conforme à l'invention, par exemple sur les lèvres, on applique une composition transparente brillante qui n'empêche pas d'observer l'effet goniochromatique et les points de surbrillance.

La composition peut comporter, en outre, des fibres goniochromatiques, afin de produire un effet visuel supplémentaire.

Outre le ou les agents de coloration goniochromatique(s) et les particules réfléchissantes, la composition peut comporter divers autres composés, et notamment au moins un agent de coloration non goniochromatique.

Cet agent de coloration non goniochromatique peut être choisi par exemple parmi les colorants, les pigments monochromes et les nacres, et peut être destiné par exemple à corriger les teintes produites par le ou les agents de coloration goniochromatiques de façon à éviter l'apparition de couleurs estimées non souhaitables. L'agent de coloration non goniochromatique peut également être présent dans la composition cosmétique pour lui conférer une couleur souhaitée dans certaines conditions d'observation.

L'invention a encore pour objet, selon un autre de ses aspects, l'utilisation d'au moins un agent de coloration goniochromatique en association avec des particules réfléchissantes pour le maquillage de la peau, des lèvres ou des phanères.

L'invention a encore pour objet l'utilisation pour le maquillage de la peau, des lèvres ou des phanères d'une composition cosmétique comportant, dans un milieu physiologiquement acceptable, au moins un agent de coloration goniochromatique, présent en une quantité allant de 2 à 15 % par rapport au poids total de la composition, et au moins des particules réfléchissantes distinctes de l'agent de coloration goniochromatique lesdites particules réfléchissantes étant des particules de verre enrobé au moins partiellement d'au moins une couche d'au moins un composé métallique, notamment un oxyde métallique.

Dans un exemple de mise en oeuvre, l'agent de coloration goniochromatique et les particules réfléchissantes sont appliqués simultanément sous la forme d'une composition telle que définie plus haut.

L'invention a encore pour objet, selon un autre de ses aspects, un procédé de maquillage d'un support choisi parmi la peau, les lèvres et les phanères, comprenant l'application consécutive sur le support d'au moins un agent de coloration goniochromatique et de particules réfléchissantes, propres à créer sur le support des points de surbrillance visibles à l'oeil nu, lesdites particules réfléchissantes étant des particules de verre enrobé au moins partiellement d'au moins une couche d'au moins un composé métallique et notamment d'un oxyde métallique.

L'invention a encore pour objet, selon un autre de ses aspects, un kit de maquillage d'un support choisi parmi la peau, les lèvres et les phanères, par la mise en oeuvre du procédé tel que décrit plus haut comportant une première et une seconde compositions cosmétiques différentes l'une de l'autre. La première composition comporte au moins des particules réfléchissantes lesdites particules réfléchissantes étant des particules de verre enrobé au moins partiellement d'au moins une couche d'au moins un composé métallique et notamment d'un oxyde métallique.. La seconde composition comporte, dans un milieu physiologiquement acceptable, au moins un agent de coloration goniochromatique. Les première et seconde compositions sont conditionnées de manière séparée.

Les caractéristiques exposées précédemment, notamment concernant la brillance moyenne, valent également pour l'une ou l'autre des première et deuxième compositions et pour la couche résultant de l'application des première et deuxième compositions.

### Exemples de particules réfléchissantes

Les particules réfléchissantes utilisées doivent être compatibles avec une utilisation en cosmétique et doivent pouvoir subsister dans le milieu physiologiquement acceptable, et notamment ne pas se dissoudre, ou en tout cas ne pas se dissoudre entièrement, dans celui-ci.

Les particules réfléchissantes peuvent être présentes dans la composition en étant dispersées de manière homogène par exemple à une teneur allant de 0,1 % à 20 % par rapport au poids total de la composition, de préférence de 1 % à 15 % en poids, et mieux de 1 % à 10 % en poids, par exemple environ 2 % notamment pour une composition destinée à être appliquée sur les lèvres. La teneur en particules réfléchissantes pourra dépendre, entre autres, de la nature du support destiné à recevoir la composition cosmétique, ainsi que de la nature du milieu physiologiquement acceptable et du ou des agents de coloration goniochromatique(s) et de la nature et de la taille des particules réfléchissantes. La teneur en particules réfléchissantes sera choisie de préférence de manière à ce que les points de surbrillance soient répartis de manière discrète sur la surface colorée goniochromatique. Les particules réfléchissantes peuvent être dans une quantité suffisante pour que l'on puisse observer simultanément, lorsque la composition cosmétique est appliquée sur un support tel que les lèvres par exemple, une pluralité de points de surbrillance, par exemple plus d'une dizaine, voire plus d'une cinquantaine, ou plus encore, par exemple plus d'une centaine ou plusieurs centaines.

Selon un mode de réalisation particulier, les particules réfléchissantes peuvent être introduites de manière telle que le rapport pondéral particules réfléchissantes/pigments goniochromatiques varie de 0,3 à 3 et en particulier de 0,5 à 2,5. En fait, ce rapport peut varier en fonction de la nature de la composition cosmétique dans laquelle sont incorporées lesdites particules. Par exemple, dans une formulation de type vernis à ongles, ce rapport pondéral particules réfléchissantes/pigments goniochromatiques peut être supérieur à 1, en particulier supérieur à 1,5 et notamment supérieur ou égal à 2. En revanche, dans des formulations de type rouges à lèvres liquides ou en bâtonnets, ce rapport pondéral peut être inférieur ou égal à 2 notamment inférieur ou égal à 1,5.

Les particules réfléchissantes peuvent être ou non goniochromatiques, interférentielles ou non, mais de préférence elles sont non goniochromatiques.

Leur taille est compatible avec la manifestation d'une réflexion spéculaire de la lumière visible (400-700 nm), d'intensité suffisante, compte tenu de la brillance moyenne de la composition, pour créer un point de surbrillance. Cette taille est susceptible de varier selon la nature chimique des particules, leur forme et leur pouvoir de réflexion spéculaire de la lumière visible.

Parmi les particules réfléchissantes utilisables dans l'invention, certaines peuvent présenter un écart relatif Δ, défini par la formule Δ= [L*_{SCI} - L*_{SCE}] / L*_{SCE}, supérieur ou égal à 0,25. Par comparaison, certaines nacres qui ne conviennent pas en tant que particules réfléchissantes présentent un coefficient Δ inférieur à 0,25. Dans la formule ci-dessus, L*_{SCI} désigne la clarté L^{*} mesurée à l'aire d'un spectrocolorimètre de marque MINOLTA et de référence CM-2002, dans un mode dit « composante spéculaire incluse », et L*_{SCE}, la clarté L^{*} mesurée à l'aide du même appareil, dans un mode dit « composante spéculaire exclue ». Pour effectuer les mesures, on réalise une dispersion à 5 % en poids des particules à tester dans un vernis à ongles transparent de composition classique (essentiellement de la nitrocellulose, une résine et un plastifiant) et l'on étale à l'état fluide une couche de 300 µm d'épaisseur de la composition ainsi formée sur le fond noir d'une carte de contraste.

On utilise la fonction SCI/SCE du spectrocolorimètre avec la géométrie d/8 pour mesurer L*_{SCI} et L*_{SCE}.

A titre d'exemple, on a mesuré pour des particules réfléchissantes de marque REFLECKS®, commercialisées par la société ENGELHARD, comportant un substrat de verre enrobé d'oxyde de fer brun, un écart relatif Δ supérieur à 0,7 alors que pour des nacres FLAMENCO® commercialisées par la même société on a mesuré un écart relatif inférieur à 0,2.

Les particules réfléchissantes présenteront de préférence une dimension d'au moins 10 µm, par exemple comprise entre environ 20 µm et environ 50 µm.

Par « dimension », on désigne la dimension donnée par la distribution granulométrique statistique à la moitié de la population, dite D50. La taille des particules réfléchissantes pourra dépendre de leur état de surface. Plus celui-ci est réfléchissant, plus la dimension pourra, a priori, être faible, et inversement.

Dans un souci d'esthétique, il est préférable que, sauf lorsqu'elles brillent pour créer des points de surbrillance, les particules réfléchissantes ne soient pas perceptibles du tout ou pas aisément perceptibles à l'oeil nu à la surface de la composition appliquée sur son support. Il est également souhaitable que les particules réfléchissantes ne soient pas de dimensions telles qu'elles créent une sensation d'inconfort sur le support. L'utilisation de particules de taille inférieure ou égale à 250 µm, et mieux inférieure ou égale à 150 µm, par exemple inférieure à 100 µm, est ainsi privilégiée. La taille des particules pourra également dépendre de la nature du support sur lequel la composition est destinée à être appliquée ; certaines parties du corps ou du visage pourront par exemple plus facilement que d'autres tolérer des dimensions plus grandes sans générer d'inconfort.

Les particules réfléchissantes peuvent présenter des formes variées. Ces particules peuvent être notamment en forme de plaquettes ou globulaires, en particulier sphériques.

Par l'expression « en forme de plaquette », on désigne des particules dont le rapport de la plus grande dimension à l'épaisseur est supérieur ou égal à 5, voire 10 ou mieux 20. L'épaisseur des particules en forme de plaquettes est par exemple comprise entre environ 0,5 µm et environ 5 µm.

Les particules présentant une surface extérieure sensiblement plane conviennent tout particulièrement, car elles peuvent plus facilement donner naissance, si leur taille, leur structure et leur état de surface le permet, à une réflexion spéculaire intense. On parle d'effet miroir.

Pour de telles particules notamment, c'est essentiellement la lumière renvoyée par réflexion dans une direction faisant, avec la normale à la surface réfléchissante, le même angle que celui que fait la lumière incidente avec cette normale, qui permet à ces particules d'apparaître comme des points de surbrillance, et non la lumière diffusée dans les autres directions.

Il peut être souhaitable que les particules réfléchissantes soient non diffusantes et non mates.

Il peut être souhaitable également que les particules réfléchissantes n'altèrent pas de manière sensible la coloration de la composition cosmétique.

A cet égard, les particules réfléchissantes qui permettent une réflexion métallique de la lumière incidente conviennent tout particulièrement. C'est le cas notamment lorsque les particules réfléchissantes permettent quelque soit leur forme, une réflexion sur une couche d'un métal, par exemple de l'argent. De telles particules s'avèrent relativement neutres vis-à-vis de la couleur de la composition.

Des particules réfléchissantes utilisables dans l'invention, à reflet métallique ou blanc, peuvent par exemple réfléchir la lumière dans toutes les composantes du visible sans absorber de manière significative une ou plusieurs longueurs d'ondes. La réflectance spectrale de ces particules réfléchissantes peut par exemple être supérieure à 70 % dans l'intervalle 400-700 nm, et mieux d'au moins 80 %, voire 90 % ou encore 95 %.

La lumière réfléchie par les particules réfléchissantes est non iridescente, notamment dans le cas d'un reflet métallique.

Les particules réfléchissantes quelque soit leur forme, présentent une structure multicouche et par exemple au moins une couche d'épaisseur uniforme d'un matériau réfléchissant.

Les particules réfléchissantes peuvent-comporter un substrat naturel ou synthétique, notamment un substrat synthétique au moins partiellement enrobé par au moins une couche d'un matériau réfléchissant.

Quelque soit la forme des particules réfléchissantes, le substrat peut, lorsqu'il est synthétique, être réalisé avec une forme favorisant la formation d'une surface réfléchissante après dépôt d'une couche de matériau réfléchissant. Le substrat peut, par exemple, présenter une surface plane et la couche de matériau réfléchissant une épaisseur sensiblement uniforme.

Le substrat peut être monomatière ou multimatériaux, plein ou creux. Le substrat peut être organique ou inorganique. Le substrat peut être naturel mais de préférence on utilise un substrat synthétique, pour la raison indiquée ci-dessus.

La couche de métal ou de composé métallique peut enrober ou non en totalité le substrat et la couche de métal peut être au moins partiellement recouverte par une couche d'un autre matériau, par exemple un matériau transparent. Il peut être préférable que la couche de métal ou de composé métallique enrobe en totalité, directement ou indirectement, c'est-à-dire avec interposition d'au moins une couche intermédiaire, métallique ou non, le substrat.

Le métal peut être choisi par exemple parmi Ag, Au, Cu, Al, Ni, Sn, Mg, Cr, Mo, Ti, Pt, Va, Rb, W, Zn, Ge, Te, Se et leurs alliages. Ag, Au, Al, Zn, Ni, Mo, Cr, Cu et leurs alliages (par exemple les bronzes et les laitons) sont des métaux préférés.

Dans le cas notamment de particules à substrat enrobé d'argent ou d'or, la couche métallique peut être présente à une teneur représentant par exemple de 0,1 à 50 % du poids total des particules, voire entre 1 et 20 %.

Des particules de verre recouvertes d'une couche métallique peuvent avoir une dimension allant par exemple de 10 µm à 300 µm, et mieux de 25 µm à 150 µm. Dans le cas où ces particules sont en forme de plaquettes, l'épaisseur peut être comprise par exemple entre environ 0,1 µm et environ 25 µm, de préférence d'environ 0,5 µm à environ 10 µm et mieux d'environ 0,5 µm à environ 5 µm. Dans le cas où ces particules se trouvent sous forme de sphères, elles peuvent avoir une dimension allant par exemple d'environ 10 à 100 µm.

Des particules de verre recouvertes d'une couche métallique sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer encore les particules comportant un substrat de borosilicate enrobé d'argent, encore appelées « nacres blanches ».

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes quelque soit leur forme, peuvent également être choisies parmi les particules à substrat synthétique enrobé au moins partiellement d'au moins une couche d'au moins un composé métallique, notamment un oxyde métallique, choisi par exemple parmi les oxydes de titane, notamment Ti02, de fer notamment Fe203, d'étain, de chrome, le sulfate de baryum et les composés suivants : MgF2, CrF3, ZnS, ZnSe, SiO2, Al2O3, MgO, Y203, Se03, SiO, HfO2, Zr02, Ce02, Nb2O5, Ta2O5, MoS2 et leurs mélanges ou alliages.
A titre d'exemple de telles particules, on peut citer par exemple les particules de verre enrobé soit d'oxyde de fer brun, soit d'oxyde de titane, d'oxyde d'étain ou d'un de leurs mélanges comme celles vendues sous la marque REFLECKS® par la société ENGELHARD.

Conviennent également à l'invention, les pigments de la gamme METASHINE 1080R commercialisée par la Société NIPPON SHEET GLASS CO. LTD. Ces pigments, plus particulièrement décrits dans la demande de brevet JP 2001-11340, sont des paillettes de verre C-GLASS comprenant 65 à 72 % de SiO₂, recouvertes d'une couche d'oxyde de titane de type rutile (TiO₂). Ces paillettes de verre ont une épaisseur moyenne de 1 micron et une taille moyenne de 80 microns soit un rapport en taille moyenne/épaisseur moyenne de 80. Elles présentent des reflets bleus, verts, jaunes ou de teinte argent selon l'épaisseur de la couche de TiO₂.

La brillance des particules réfléchissantes peut être encore due, en variante où additionnellement, à la réflexion de la lumière sur une couche d'un matériau de la particule présentant un indice de réfraction suffisant grand par rapport à celui du milieu d'où provient la lumière incidente.

La composition cosmétique selon l'invention peut bien entendu comporter des particules réfléchissantes de différentes natures sans que l'on sorte du cadre de la présente invention.

### Exemples d'agents de coloration goniochromatiques

La composition contient un ou plusieurs agents de coloration goniochromatiques pour créer, lorsque la composition est appliquée sur son support un fond coloré dont la couleur change avec l'angle d'observation et avec lequel les particules réfléchissantes contrastent. On peut utiliser un seul agent de coloration goniochromatique pour une facilité de mise en oeuvre.

L'agent de coloration goniochromatique doit être présent en une quantité pouvant varier, en poids par rapport au poids total de la composition, de 2 à 15 %, et mieux de 2 à 10 % notamment pour une composition destinée à être appliquée sur les lèvres. Dans le cas d'une telle composition, des résultats très satisfaisants ont pu être obtenus pour une teneur d'agent de coloration goniochromatique comprise entre 2 et 8 % combinée à une teneur en particules réfléchissantes comprise entre 1 et 5 %, en poids. Une composition de vernis à ongles pourra contenir par exemple 5% d'agent de coloration goniochromatique; un fond de teint pourra en contenir de 10 à 15% et un rouge à lèvres pourra en contenir de 2 à 8 % en poids.

L'agent de coloration goniochromatique peut être choisi de manière à présenter un changement de couleur relativement important avec l'angle d'observation.

L'agent de coloration goniochromatique peut ainsi être choisi de telle sorte que l'on puisse observer, pour une variation de l'angle d'observation comprise entre 0° et 80° sous un éclairage à 45°, une variation de couleur ΔE de la composition cosmétique, mesurée dans l'espace colorimétrique CIE 1976, d'au moins 2.

L'agent de coloration goniochromatique peut également être choisi de telle sorte que l'on puisse observer, pour un éclairage à 45° et une variation de l'angle d'observation comprise entre 0° et 80°, une variation Dh de l'angle de teinte de la composition cosmétique, dans le plan CIE 1976, d'au moins 30° voire au moins 40° ou au moins 60°, voire encore d'au moins 100°.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

La structure multicouche peut présenter ou non, par rapport à une couche centrale, une symétrie au niveau de la nature chimique des couches empilées.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃; TiO₂/SiO₂/TiO₂; TiO₂/Al₂O₃/TiO₂; SnO/TiO₂/SiO₂/TiO₂/SnO; Fe₂O₃/SiO₂/Fe₂O₃ et SnO/mica/TiO₂/SiO₂/TiO₂/mica/SnO, des pigments ayant ces structures étant commercialisés sous la dénomination XIRONA par la société MERCK (Darmstadt). A titre illustratif de ces pigments, on peut plus particulièrement citer les pigments de structure silice/oxyde de titane/oxyde d'étain commercialisés sous le nom XIRONA MAGIC par la Société MERCK, les pigments de structure silice/oxyde de fer brun commercialisés sous le nom XIRONA INDIAN SUMMER par la Société MERCK et les pigments de structure silice/oxyde de titane/mica/oxyde d'étain commercialisés sous le nom XIRONA CARRIBEAN BLUE par la Société MERCK. On peut encore citer les pigments INFINITE COLORS de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut encore utiliser des agents de coloration goniochromatiques à structure multicouche comprenant une alternance de couches polymériques par exemple du type naphtalate de polyéthylène et téréphtalate de polyéthylène. De tels agents sont notamment décrits dans WO-A-96/19347 et WO-A-99/36478.

On peut citer, à titre d'exemple de pigments à structure multicouche polymérique, ceux commercialisés par la société 3M sous la dénomination COLOR GLITTER.

Les agents de coloration à cristaux liquides comprennent par exemple des silicones ou des éthers de cellulose sur lesquels sont greffés des groupes mésomorphes.

Comme particules goniochromatiques à cristaux liquides, on peut utiliser par exemple celles vendues par la société CHENIX ainsi que celle commercialisées sous la dénomination HELICONE® HC par la société WACKER.

La composition peut en outre comporter des fibres goniochromatiques dispersées. De telles fibres peuvent par exemple présenter une taille comprise entre 200 µm et 700 µm, par exemple d'environ 300 µm.

En particulier, on peut utiliser des fibres interférentielles à structure multicouche. Des fibres à structure multicouche de polymères sont notamment décrites dans les documents EP-A-921217, EP-A-686858 et US-A-5472798. La structure multicouche peut comporter au moins deux couches, chaque couche, indépendamment ou non de la (ou les) autre(s) couche(s), étant réalisée en au moins un polymère de synthèse. Les polymères présents dans les fibres peuvent avoir un indice de réfraction allant de 1,30 à 1,82 et mieux allant de 1,35 à 1,75. Les polymères préférés pour constituer les fibres sont les polyesters tels que le polyéthylène téréphtalate, le polyéthylène naphtalate, le polycarbonate ; les polymères acryliques comme le polyméthacrylate de méthyle ; les polyamides.

Des fibres goniochromatiques à structure bicouche polyéthylène téréphtalate/nylon-6 sont commercialisées par la société TEIJIN sous la dénomination MORPHOTEX.

### Base brillante

La composition peut également comprendre au moins un composé capable de lui conférer de la brillance et notamment une phase huileuse, en particulier une phase huileuse présentant un indice de réfraction compris entre 1,47 et 1,51, mieux entre 1,48 et 1,50. L'indice de réfraction est mesuré à température ambiante (25°C), à l'aide d'un refractomètre.

Une telle phase huileuse peut s'avérer utile dans le cas d'un gloss liquide notamment.

Selon un exemple de mise en oeuvre de l'invention, on choisit une base brillante telle que décrite dans la demande EP-A-792 637.

La composition cosmétique peut contenir par exemple au moins une huile d'origine minérale, végétale ou synthétique, carbonés, hydrocarbonée, fluorée et/ou siliconée.

Par « huile hydrocarbonée », on entend des huiles contenant majoritairement des atomes de carbone et des atomes d'hydrogène et en particulier des chaînes alkyle ou alcényle comme les alcanes ou alcènes mais aussi les huiles à chaîne alkyle ou alcényle comportant un ou des groupements alcool, éther, ester et/ou acide carboxylique.

Comme huiles utilisables, on peut ainsi citer, cette liste n'étant pas limitative, les huiles hydrocarbonées d'origine minérale ou synthétique telles les hydrocarbures linéaires ou ramifiés comme l'huile de paraffine ou ses dérivés, l'huile de vaseline, les polydécènes, le polyisobutène hydrogéné tel que le Parféam^{®} commercialisé par la société Nippon Oil Fats, le squalane d'origine synthétique ou végétale; les huiles d'origine animale comme l'huile de vison, de tortue, le perhydrosqualène; les huiles d'origine végétale hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées, comme l'huile d'amande douce, de calophyllum, de palme, de pépins de raisin, de sésame, d'arara, de colza, de tournesol, de coton, d'abricot, de ricin, de luzerne, de courge, dé cassis, de macadamia, de rosier muscat, de noisette, d'avocat, de jojoba, d'olive ou de germes de céréales (maïs, blé, orge, seigle) ; des esters d'acide gras et notamment d'acide lanolique, d'acide oléique, d'acide laurique, d'acide stéarique ; les esters de synthèse, comme par exemple l'huile de Purcellin (octanoate de cétostéaryle), l'isononanoate d'isononyle, le benzoate d'alcool en C₁₂ à C₁₅, le palmitate d'éthyl 2-hexyle, des octanoates, décanoates ou ricinoléates d'alcools ou de polyalcools, le myristate d'isopropyle, le palmitate d'isopropyle, le stéarate de butyle, le laurate d'hexyle, l'adipate de diisopropyle, le palmitate de 2-éthyl-hexyle, le laurate de 2-hexyl-décyle, le palmitate de 2-octyl-décyle, le myristate de 2-octyl-dodécyle, le succinate de 2-diéthyl-hexyle, le malate de diisostéaryle, le triisostéarate de glycérine ou de diglycérine ; les esters hydroxylés comme le lactate d'isostéaryle ; les esters du pentaérythritol ; les acides gras supérieurs en C₈-C₂₆ tels que l'acide oléique, l'acide linoléique, l'acide linolénique ou l'acide isostéarique ; les alcools gras supérieurs en C₈-C₂₆ tels que l'alcool oléique, l'alcool linoléique ou linolénique, l'alcool isostéarique ou l'octyl dodécanol ; les éthers de synthèse à au moins 7 atomes de carbone, les huiles siliconées telles que les polydiméthylsiloxanes (PDMS) liquides à température ambiante, linéaires, éventuellement phénylés tels que les phényltriméthicones, les phényl triméthylsiloxy diphénylsiloxanes, les diphényl diméthicones, les diphényl méthyldiphényl trisiloxanes, les 2-phényléthyl triméthylsiloxysilicates liquides, éventuellement substitués par des groupements aliphatiques et/ou aromatiques comme les groupes alkyle, alkoxy ou phényle, pendant et/ou en bout de chaîne siliconée, groupements ayant de 2 à 24 atomes de carbone et éventuellement fluorés, ou par des groupements fonctionnels tels que des groupements hydroxyle, thiol et/ou amine ; les polysiloxanes modifiés par des acides gras, des alcools gras ou des polyoxyalkylènes comme les diméthicones copolyols ou les alkylméthicones copolyols ; les silicones fluorées liquides ; ou encore les triglycérides des acides caprylique/caprique comme ceux vendus par la société Stearineries Dubois ou ceux vendus sous les dénominations Miglyol 810, 812 et 818 par la société Dynamit Nobel ; et leurs mélanges.

Une bonne dispersion des pigments et/ou des charges, dans la composition cosmétique, peut également permettre d'améliorer la brillance de la couche de composition appliquée sur son support.

Dans le cas d'un vernis à ongle, la brillance peut être obtenue en introduisant dans la composition du vernis des composés par exemple de type polyuréthane et latex.

### Agents de coloration non goniochromatiques

La composition cosmétique peut incorporer un ou plusieurs agents de coloration non goniochromatiques et ne constituant pas des particules réfléchissantes, par exemple choisis parmi les colorants, notamment liposolubles ou hydrosolubles, les pigments monochromes, et les nacres classiquement utilisées dans les compositions cosmétiques.

Comme colorants utilisables, on peut citer par exemple le rouge Soudan, le DC Red 17, le DC Green 6, le β-carotène, l'huile de soja, le brun Soudan, le DC Yellow 11, le DC Violet 2, le DC orange 5, le jaune quinoléine, le rocou, les dérivés caroténoïdes comme le lycopène, le béta-carotène, la bixine, la capsantéine, et/ou leurs mélanges, ces colorants étant liposolubles. Des colorants hydrosolubles tels que par exemple le sulfate de cuivre, de fer, des sulfopolyesters hydrosolubles tels que ceux décrits dans la demande FR-96 154 152, les rhodamines, les colorants naturels (carotène, jus de betterave), le bleu de méthylène, le caramel peuvent également être utilisés.

Les colorants peuvent représenter par exemple de 0,01 à 20 % du poids total de la composition et mieux de 0,1 à 10 %.

Comme pigments susceptibles d'être utilisés, on peut citer les pigments constitués par des particules blanches ou colorées, destinés par exemple à colorer et/ou opacifier la composition. Parmi les pigments utilisables, on peut citer le noir de carbone, les laques de baryum, strontium, calcium, aluminium, les oxydes de titane, de zirconium ou de cérium, de zinc, de fer ou de chrome et le bleu ferrique.

Des nacres peuvent être présentes dans la composition à raison par exemple de 0 à 20 % du poids total de la composition, voire à un taux de l'ordre de 1 à 15 %. A titre d'exemples de nacres, on peut citer le mica naturel recouvert d'oxyde de titane, d'oxyde de fer, de pigment naturel ou d'oxychlorure de bismuth. Parmi les nacres disponibles sur le marché, on peut citer les nacres TIMICA et FLAMENCO commercialisées par la société ENGELHARD et les nacres TIMIRON commercialisées par MERCK.

Les agents de coloration non goniochromatiques peuvent représenter par exemple de 0,001 à 60 % du poids total de la composition, de préférence de 0,01 à 50 % et mieux de 0,1 à 40%. Pour des compositions pulvérulentes, la quantité d'agents de coloration peut aller jusqu'à 85 % et même jusqu'à 98 %.

### Milieu physiologiquement acceptable

Le milieu physiologiquement acceptable sera adapté à la nature du support sur lequel doit être appliqué la composition ainsi qu'à la forme sous laquelle la composition est destinée à être conditionnée, notamment solide ou fluide à température ambiante et pression atmosphérique.

La composition selon l'invention peut comprendre un milieu cosmétique aqueux et/ou une phase grasse.

La composition peut comprendre de l'eau ou un mélange d'eau et de solvants organiques hydrophiles comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La phase hydrophile peut, en outre, contenir des éthers en C₂ et des aldéhydes en C₂-C₄ hydrophiles. L'eau ou le mélange d'eau et de solvants organiques hydrophiles peut être présent dans la composition selon l'invention, ou l'une des compositions de base et/ou de surface, en une teneur allant de 0 % à 90 % (notamment 0,1 % à 90 %) en poids, par rapport au poids total de la composition, et de préférence de 0 % à 60 % en poids (notamment 0,1 % à 60 % en poids).

La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Cette phase grasse peut, en outre, contenir des solvants organiques lipophiles.

Comme corps gras liquides à température ambiante, appelés souvent huiles, utilisables dans l'invention, on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyltrisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

La composition de l'invention peut en outre avantageusement comprendre un corps gras solide ou pâteux à température ambiante, comme les gommes ou les cires. Les cires peuvent être hydrocarbonées, fluorées et/ou siliconées et être d'origine végétale, minérale, animale et/ou synthétique. En particulier, les cires peuvent présenter une température de fusion supérieure à 25 °C et mieux supérieure à 45 °C.

Comme cires utilisables dans une composition selon l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes pouvant être utilisées sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. La composition peut contenir de 0 à 50 % en poids de cires, par rapport au poids total de la composition et mieux de 1 à 30 % en poids.

La composition selon l'invention peut également comprendre un ou plusieurs solvants organiques, physiologiquement acceptables. Ces solvants peuvent être présents en une teneur allant de 0 à 90 %, mieux de 0 à 60% en poids, par rapport au poids total de la composition et encore mieux de 0,1 à 30 %.

La présence de solvants organiques convient plus particulièrement pour le maquillage des ongles. La composition constitue alors généralement un vernis à ongles. Le solvant organique peut être présent dans la composition cosmétique à une teneur allant par exemple de 30 à 99 % en poids, par rapport au poids total de la composition, et de préférence de 60 % à 90 % en poids.

Lorsque le milieu physiologiquement acceptable de la composition contient une phase liquide, cette phase peut être notamment une phase organique liquide dans laquelle de l'eau est dispersée ou émulsionnée.

La composition peut présenter une phase grasse continue, pouvant contenir moins de 5% d'eau, notamment moins de 1% d'eau par rapport à son poids total et en particulier être sous forme anhydre.

### Charges

La composition cosmétique peut comprendre en outre des charges.

Par « charges », on désigne des particules de toute forme insolubles dans le milieu de la composition quelle que soit la température à laquelle la composition est fabriquée. Ces charges peuvent servir notamment à modifier la rhéologie ou la texture de la composition.

A titre d'exemple de charges, on peut citer, entre autres, le talc, le mica, la silice, le kaolin, et les poudres de polyamide (Nylon®) (Orgasol® de chez Atochem).

### Agents actifs cosmétiques

La composition cosmétique peut également contenir un ou plusieurs actifs cosmétiques, dermatologiques, hygiéniques ou pharmaceutiques.

Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...). Ces actifs peuvent être utilisés par exemple à des concentrations de 0 à 20 % et notamment de 0,001 à 15 % par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants les filtres UV, ou leurs mélanges.

La composition cosmétique peut, de plus, comprendre, selon le type d'application envisagée, les constituants classiquement utilisés dans les domaines considérés, qui sont présents en une quantité appropriée à la forme galénique souhaitée.

La composition cosmétique peut se présenter sous toute forme galénique normalement utilisée pour une application topique et notamment sous forme anhydre, sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules situés à l'interface huile/eau.

La composition de l'invention peut être sous forme de poudre, de liquide, de solide ou de semi-solide, notamment de produit coulé en stick ou en coupelle, de bâtonnet, de pâte ou de crème plus ou moins fluide.

La composition cosmétique peut constituer, entre autres, un rouge à lèvres, un gloss liquide, une pâte de rouge à lèvres, un fard à joues, un crayon à lèvres, un fond de teint solide ou fluide, un produit anti-cernes ou de contour des yeux, un eye-liner, un mascara, un vernis à ongles, une ombre à paupières, un produit de maquillage du corps ou des cheveux ou encore un produit solaire ou de coloration de la peau.

La composition de l'invention peut être obtenue selon les procédés de préparation classiquement utilisés en cosmétique.

Les exemples soumis ci-après sont présentés à titre illustratif et non limitatif de l'invention.

### Exemples

Les pourcentages ci-après sont tous exprimés en poids, par rapport au poids total de la composition.

L'invention s'applique tout particulièrement aux compositions destinées à être appliquées sur les lèvres.

Pour vérifier les propriétés optiques d'une composition réalisée conformément à l'invention, on a réalisé un gloss liquide ayant la composition suivante.

### Gloss liquide

| | |
|---|---|
| Polyacryladipate-2 de bis-diglycéryle | 17,5 |
| Malate de diisostéraryle | 9,5 |
| Trimellitate de tridécyle | 10 |
| Triglycéride d'acide C18-36 | 19 |
| Silice diméthyl silylate | 8 |
| Particules de verre enrobé d'argent (METASHINE®)* | 2 |
| Pigment goniochromatique (SICOPEARL®)** | 5 |
| Nacre | 3 |
| Polybutène | 12 |
| Tétraisostéarate de pentaerythrityle | 13 |
| Parfum, conservateur | qs |

| | |
|---|---|
| * commercialisées par la société TOYAL | |
| ** commercialisé par la société BASF | |

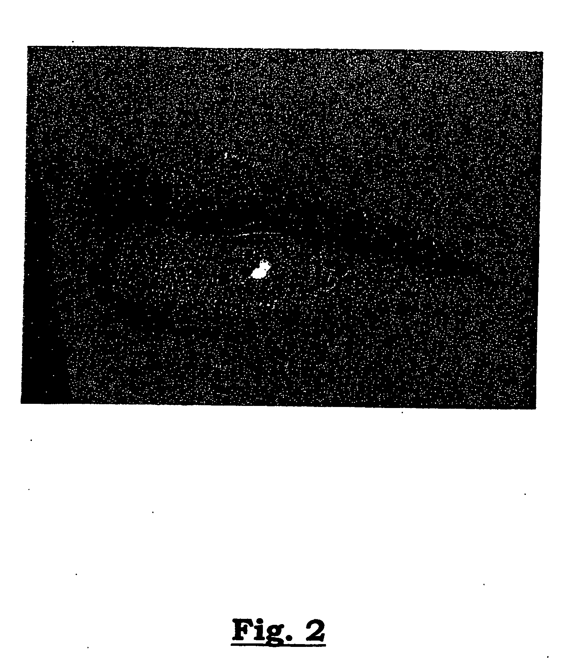

Après application, on peut constater que la composition rend les lèvres plus « pulpeuses ». La figure 2 est une photographie des lèvres maquillées. On peut remarquer sur la photographie la présence de nombreux points de surbrillance et la brillance moyenne élevée de la composition.

On a également réalisé un rouge à lèvres semi-solide.

### Rouge à lèvres en bâtonnet

| | |
|---|---|
| Néopentanoate d'octyldodécyle | 17,0 |
| Triglycéride d'acide caprique/caprylique | 10,2 |
| Huile de lanoline | 15,0 |
| Lanoline acétylée | 10,2 |
| Polybutène | 15,0 |
| Particules de verre enrobé d'argent (METASHINE® REFSX) | 4,0 |
| Pigment goniochromatique (SICOPEARL®) | 3,0 |
| Cire microcristalline | 2,5 |
| Cire de polyéthylène | 7,4 |
| Phényl triméthicone | 7,0 |
| Polyisobutène hydrogéné | 6,5 |
| Parfum, conservateur, antioxydant | qs |

Mode opératoire : On disperse la bentone dans une partie de la phase huileuse, puis on ajoute le reste de la phase grasse que l'on chauffe à 95°C. Après homogénéisation et broyage des pigments, le mélange est coulé dans des moules adéquats.

On obtient des bâtonnets de rouge à lèvres donnant après application sur ces dernières une impression de volume. La composition présente également de bonnes propriétés d'application.

Bien entendu, l'invention n'est pas limitée aux exemples de mise en oeuvre qui viennent d'être décrits.

On peut notamment réaliser, conformément à l'invention, une composition comportant des agents de coloration goniochromatiques de différentes natures ainsi que des particules réfléchissantes de différentes natures.

Dans toute la description, y compris les revendications, l'expression « comportant un » doit être comprise comme étant synonyme de « comportant au moins un », sauf si le contraire est spécifié.

## Revendications

1. Composition cosmétique comportant, dans un milieu physiologiquement acceptable, au moins un agent de coloration goniochromatique, présent en une quantité allant de 2 à 15 % par rapport au poids total de la composition, et au moins des particules réfléchissantes distinctes de l'agent de coloration goniochromatique lesdites particules réfléchissantes étant des particules de verre enrobé au moins partiellement d'au moins une couche d'au moins un composé métallique, notamment un oxyde métallique.

2. Composition selon la revendication 1, **caractérisée par le fait que** le composé métallique est choisi parmi les oxydes de titane, notamment TiO₂, de fer, d'étain, notamment Fe₂O₃, de chrome, le sulfate de baryum et les composés MgF₂, CeF₃, ZnS, ZnSe, SiO₂, Al₂O₃, MgO, Y₂O₃, SeO₃, SiO, HfO₂, ZrO₂, CeO₂, Nb₂O₅, Ta₂O₅ et MoS₂ et leurs mélanges.

3. Composition selon la revendication 2, **caractérisée par le fait que** le composé métallique est un oxyde de titane, un oxyde de fer ou un oxyde d'étain ou un de leurs mélanges.

4. Composition selon l'une quelconque des revendications précédentes, **caractérisée par le fait que** la brillance moyenne de la composition est supérieure ou égale à 30.

5. Composition selon la revendication 4, **caractérisée par le fait que** sa brillance moyenne est supérieure ou égale à 50.

6. Composition selon la revendication 5, **caractérisée par le fait que** sa brillance moyenne est supérieure ou égale à 70.

7. Composition selon l'une quelconque des revendications précédentes, **caractérisée par le fait que** l'agent de coloration goniochromatique est choisi de telle sorte que l'on puisse observer sur la couche de composition cosmétique, pour un éclairage à 45° et une variation de l'angle d'observation comprise entre 0° et 80°, une variation Dh de l'angle de teinte de la composition cosmétique d'au moins 30°.

8. Composition selon l'une quelconque des revendications précédentes, **caractérisée par le fait que** l'agent de coloration goniochromatique est choisi de telle sorte que l'on puisse observer sur la couche de composition cosmétique, pour un éclairage à 45° et une variation de l'angle d'observation comprise entre 0° et 80°, une variation Dh de l'angle de teinte de la composition cosmétique d'au moins 60°.

9. Composition selon l'une quelconque des revendications précédentes, **caractérisée par le fait que** les particules réfléchissantes sont configurées pour présenter une réflectance spectrale dans le spectre visible d'au moins 70 %.

10. Composition selon l'une quelconque des revendications précédentes, **caractérisée en ce que** les particules réfléchissantes présentent une dimension au plus égale à 250 µm, de préférence inférieure à 150 et mieux 100 µm.

11. Composition selon l'une quelconque des revendications précédentes, **caractérisée par le fait que** les particules réfléchissantes présentent une dimension au moins égale à 10 µm.

12. Composition selon l'une quelconque des revendications précédentes, **caractérisée par le fait que** les particules réfléchissantes présentent une dimension comprise entre 20 µm et 50 µm.

13. Composition selon l'une quelconque des revendications précédentes, **caractérisée en ce que** les particules réfléchissantes sont présentes dans la composition à une teneur allant de 0,1 % à 20 %, en particulier de 1 à 15 %, notamment de 1 à 10 % par rapport au poids total de la composition.

14. Composition selon l'une quelconque des revendications précédentes, **caractérisée par le fait que** les particules réfléchissantes se présentent sous la forme de plaquettes, ou de sphères.

15. Composition selon l'une quelconque des revendications précédentes, **caractérisée en ce que** l'agent de coloration goniochromatique est choisi parmi les agents de coloration à cristaux liquides et les structures multicouche interférentielles.

16. Composition selon l'une quelconque des revendications précédentes, **caractérisée en ce que** l'agent de coloration goniochromatique comporte une structure multicouche interférentielle choisie parmi les structures suivantes : Al/SiO₂/Al//SiO₂/Al ; Cr/MgF₂/Al/MgF₂/Cr ; MoS₂/SiO₂/Al/SiO₂/MoS₂ ; Fe₂O₃/SiO₂/Al/SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ Fe₂O₃/SiO₂/mica-oxyde/SiO₂/Fe₂O₃ ; TiO₂/SiO₂/TiO₂ ; TiO₂/Al₂O₃/TiO₂ ; SnO/TiO₂/SiO₂/TiO₂/SnO ; Fe₂O₃/SiO₂/Fe₂O₃ et SnO/mica/TiO₂/SiO₂/SiO₂/ToO₂/mica/SnO.

17. Composition selon la revendication 16, **caractérisée en ce que** l'agent de coloration goniochromatique comporte une structure multicouche interférentielle choisie parmi les structures suivantes : MoS₂/SiO₂/Al/SiO₂/MoS₂ ; Fe₂O₃/SiO₂/Al/SiO₂/Fe₂O₃ ; Fe₂O₃/SiO₂/Fe₂O₃/SiO₂/Fe₂O₃ ; SnO/TiO₂/SiO₂/TiO₂/SnO; Fe₂O₃/SiO₂/Fe₂O₃ et SnO/mica/TiO₂/SiO₂/TiO₂/mica/SnO.

18. Composition selon l'une quelconque des revendications précédentes, **caractérisée en ce que** l'agent de coloration goniochmmatique est présent en une quantité allant de 0,1 1 à 20 %, mieux de 2 à 10 % par rapport au poids total de la composition.

19. Composition selon l'une quelconque des revendications précédentes, **caractérisée en ce qu'**elle comporte une base brillante, de brillance moyenne supérieure à 20.

20. Composition selon la revendication 19, **caractérisée par le fait qu'**elle comporte une base brillante, de brillante moyenne supérieure à 50.

21. Composition selon la revendication 20, **caractérisée par le fait qu'**elle comporte une base brillante, de brillance moyenne supérieure à 70.

22. Composition selon l'une quelconque des revendications précédentes, **caractérisée en ce qu'**elle comprend en outre au moins un agent de coloration non goniochromatique.

23. Composition selon la revendication 22, **caractérisée par le fait que** l'agent de coloration non goniochromatique est choisi parmi les colorants, les pigments monochromes et les nacres.

24. Composition selon l'une quelconque des revendications précédentes, **caractérisée par le fait qu'**elle comprend des fibres goniochromatiques.

25. Composition selon l'une quelconque des revendications précédentes, **caractérisée en ce qu'**elle se présente sous l'une des formes suivantes : sous forme anhydre, sous forme d'une solution huileuse ou aqueuse, d'un gel huileux ou aqueux, d'une émulsion huile dans eau ou eau dans huile, d'une émulsion multiple, d'une dispersion d'huile dans de l'eau grâce à des vésicules situés à l'interface huile/eau.

26. Composition selon l'une quelconque des revendications précédentes, **caractérisée par le fait qu'**elle se présente sous la forme d'un gloss liquide.

27. Produit pour le maquillage des lèvres comportant une composition telle que définie dans l'une quelconque des revendications précédentes.

28. Vernis à ongles comportant une composition telle que définie dans l'une quelconque des revendications 1 à 25.

29. Fond de teint comportant une composition telle que définie dans l'une quelconque des revendications 1 à 25.

30. Mascara comportant une composition telle que définie dans l'une quelconque des revendications 1 à 25.

31. Utilisation pour le maquillage de la peau, des lèvres ou des phanères d'une composition selon l'une quelconque des revendications 1 à 30.

32. Procédé de maquillage d'un support choisi parmi la peau, les lèvres et les phanères, comprenant l'application consécutive sur le support d'au moins un agent de coloration goniochromatique et de particules réfléchissantes, propres à créer sur le support des points de surbrillance visibles à l'oeil nu, lesdites particules réfléchissantes étant des particules de verre enrobé au moins partiellement d'au moins une couche d'au moins un composé métallique et notamment d'un oxyde métallique.

33. Kit de maquillage d'un support choisi parmi la peau, les lèvres et les phanères, pour la mise en oeuvre du procédé selon la revendication 32, comportant une première et une seconde compositions cosmétiques différentes l'une de l'autre, la première composition comportant au moins des particules réfléchissantes, lesdites particules réfléchissantes étant des particules de verre enrobé au moins partiellement d'au moins une couche d'au moins un composé métallique et notamment d'un oxyde métallique, la seconde composition comportant, dans un milieu physiologiquement acceptable, au moins un agent de coloration goniochromatique, les première et seconde compositions étant conditionnées de manière séparée.

## Claims

1. Cosmetic composition comprising, in a physiologically acceptable medium, at least one goniochromatic colouring agent, present in an amount ranging from 2% to 15% relative to the total weight of the composition, and at least some reflective particles that are different from the goniochromatic colouring agent, the said being reflective particles of particles glass coated at least partially with at least one layer of at least one metallic compound and especially a metal oxide.

2. Composition according to Claim 1, **characterized in that** the metallic compound is chosen from titanium oxides, especially TiO₂, iron oxides, tin oxide, especially Fe₂O₃, chromium oxide, barium sulphate and the compounds MgF₂, CeF3, ZnS, ZnSe, SiO₂, Al₂O₃, MgO, Y₂O₃, SeO₃, SiO, HfO₂, ZrO₂, CeO₂, Nb₂O₅, Ta₂O₅ and MoS₂, and mixtures thereof.

3. Composition according to Claim 2, **characterized in that** the metallic compound is a titanium oxide, an iron oxide or a tin oxide, or a mixture thereof.

4. Composition according to any one of the preceding claims, **characterized in that** the mean gloss of the composition is greater than or equal to 30.

5. Composition according to Claim 4, **characterized in that** its mean gloss is greater than or equal to 50.

6. Composition according to Claim 5, **characterized in that** its mean gloss is greater than or equal to 70.

7. Composition according to any one of the preceding claims, **characterized in that** the goniochromatic colouring agent is chosen such that a variation Dh of the hue angle of the cosmetic composition of at least 30° may be observed on the layer of cosmetic composition, for an illumination at 45° and a variation of the angle of observation of between 0° and 80°.

8. Composition according to any one of the preceding claims, **characterized in that** the goniochromatic colouring agent is chosen such that a variation Dh of the hue angle of the cosmetic composition of at least 60° may be observed on the layer of cosmetic composition for an illumination at 45° and a variation of the angle of observation of between 0° and 80°.

9. Composition according to any one of the preceding claims, **characterized in that** the reflective particles are configured to have a spectral reflectance in the visible spectrum of at least 70%.

10. Composition according to any one of the preceding claims, **characterized in that** the reflective particles are not more than 250 µm, preferably less than 150 and better still 100 µm in size.

11. Composition according to any one of the preceding claims, **characterized in that** the reflective particles are at least 10 µm in size.

12. Composition according to any one of the preceding claims, **characterized in that** the reflective particles are between 20 µm and 50 µm in size.

13. Composition according to any one of the preceding claims, **characterized in that** the reflective particles are present in the composition in a content ranging from 0.1% to 20%, in particular from 1% to 15% and especially from 1% to 10% relative to the total weight of the composition.

14. Composition according to any one of the preceding claims, **characterized in that** the reflective particles are in the form of platelets or spheres.

15. Composition according to any one of the preceding claims, **characterized in that** the goniochromatic colouring agent is chosen from liquid-crystal colouring agents and multilayer interference structures.

16. Composition according to any one of the preceding claims, **characterized in that** the goniochromatic colouring agent comprises a multilayer interference structure chosen from the following structures: Al/SiO₂/Al/SiO₂/Al; Cr/MgF₂/Al/MgF₂/Cr; MoS2/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃; MoS₂/SiO₂/mica-oxide/SiO₂/MoS₂; Fe₂O₃/SiO₂/mica-oxide/SiO₂/Fe₂O₃, TiO₂/SiO₂/TiO₂; TiO₂/Al₂O₃/TiO₂;

17. Composition according to claim 16, **characterized in that** the goniochromatic colouring agent comprises a multilayer interference structure chosen from the following structures: MoS₂/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃; SnO/TiO₂/SiO₂/TiO₂/SnO; Fe₂O₃/SiO₂/Fe₂O₃ and SnO/mica/TiO₂/SiO₂/TiO₂/mica/SnO.

18. Composition according to any one of the preceding claims, **characterized in that** the goniochromatic colouring agent is present in an amount ranging from 0.1% to 20%, better still from 2% to 10% relative to the total weight of the composition.

19. Composition according to any one of the preceding claims, **characterized in that** it comprises a gloss base, with a mean gloss of greater than 20.

20. Composition according to Claim 19, **characterized in that** it comprises a gloss base with a mean gloss of greater than 50.

21. Composition according to Claim 20, **characterized in that** it comprises a gloss base with a mean gloss of greater than 70.

22. Composition according to any one of the preceding claims, **characterized in that** it also comprises at least one non-goniochromatic colouring agent.

23. Composition according to Claim 22, **characterized in that** the non-goniochromatic colouring agent is chosen from dyes, monochromatic pigments and nacres.

24. Composition according to any one of the preceding claims, **characterized in that** it comprises goniochromatic fibres.

25. Composition according to any one of the preceding claims, **characterized in that** it is in one of the following forms: in anhydrous form, in the form of an oily or aqueous solution, an oily or aqueous gel, an oil-in-water or water-in-oil emulsion, a multiple emulsion, a dispersion of oil in water by means of vesicles located at the oil/water interface.

26. Composition according to any one of the preceding claims, **characterized in that** it is in the form of a liquid gloss.

27. Lip makeup product comprising a composition as defined in any one of the preceding claims.

28. Nail varnish comprising a composition as defined in any one of Claims 1 to 25.

29. Foundation comprising a composition as defined in any one of Claims 1 to 25.

30. Mascara comprising a composition as defined in any one of Claims 1 to 25.

31. Use for making up the skin, the lips or the integuments, of a composition according to any one of claims 1 to 30.

32. Process for making up a support chosen from the skin, the lips and the integuments, comprising the consecutive application to the support of at least one goniochromatic colouring agent and of reflective particles capable of creating the support highlighten points that are visible to the naked eye, the said reflective particles being particles of glass.

33. Kit for making up a support chosen from the skin, the lips and the integuments, comprising a first and a second cosmetic composition that are different from each other, for implementing the process according to claim 32, the first composition comprising at least some reflective particles, the said reflective particles being particles of glass coated at least partially with at least one layer of at least one metallic compound and especially -a metal oxide, the second composition comprising at least one goniochromatic colouring agent in a physiologically acceptable medium, the first and second compositions being packaged separately.

## Patentansprüche

1. Kosmetische Zusammensetzung, umfassend, in einem physiologisch verträglichen Medium mindestens ein goniochromatisches Farbmittel, das in einer Menge von 2-15 %, bezogen auf das Gesamtgewicht der Zusammensetzung vorhanden ist, und mindestens distinkte reflektierende Teilchen des goniochromatischen Farbmittels, wobei die reflektierenden Teilchen Teilchen aus Glas sind, die mindestens teilweise von mindestens einer Schicht von mindestens einer Metallverbindung, insbesondere einem Metalloxid überzogen sind.

2. Zusammensetzung nach Anspruch 1, **dadurch gekennzeichnet, dass** die Metallverbindung aus Oxiden von Titan, insbesondere TiO₂, von Eisen, von Zinn, insbesondere Fe₂O₃, von Chrom, Bariumsulfat und den Verbindungen MgF₂, CeF₃, ZnS, ZnSe, SiO₂, Al₂O₃, MgO, Y₂O₃, SeO₃, SiO, HfO₂, ZrO₂, CeO₂, Nb₂O₅, Ta₂O₅ und MoS₂ und ihren Gemischen ausgewählt ist.

3. Zusammensetzung nach Anspruch 2, **dadurch gekennzeichnet, dass** die Metallverbindung ein Oxid von Titan, ein Oxid von Eisen oder ein Oxid von Zinn oder eines von ihren Gemischen ist.

4. Zusammensetzung nach eine der vorhergehenden Ansprüche, **dadurch gekennzeichnet, dass** die durchschnittliche Glanzzahl der Zusammensetzung größer oder gleich 30 ist.

5. Zusammensetzung nach Anspruch 4, **dadurch gekennzeichnet, dass** ihre durchschnittliche Glanzzahl größer oder gleich 50 ist.

6. Zusammensetzung nach anspruch 5, **dadurch gekennzeichnet, dass** ihre durchschnittliche Glanzzahl größer oder gleich 70 ist.

7. Zusammensetzung nach einem der vorhergehenden Ansprüche, **dadurch gekennzeichnet, dass** das goniochromatische Farbmittel so gewählt ist, dass auf der Schicht von kosmetischer Zusammensetzung für eine Beleuchtung bei 45 ° für eine Variierung des Beobachtungswinkels zwischen 0 ° 80 ° eine Variierung Dh des Farbwinkels der kosmetischen Zusammensetzung von mindestens 30 ° festgestellt werden kann.

8. Zusammensetzung nach einem der vorhergehenden Ansprüche, **dadurch gekennzeichnet, dass** das goniochromatische Farbmittel so gewählt ist, dass auf der Schicht von kosmetischer Zusammensetzung für eine Beleuchtung bei 45 ° und für eine Variierung des Beobachtungswinkels zwischen 0 ° und 80 ° eine Variierung Dh des Farbwinkels der kosmetischen Zusammensetzung von mindestens 60 ° festgestellt werden kann.

9. Zusammensetzung nach einem der vorhergehenden Ansprüche, **dadurch gekennzeichnet, dass** die reflektierenden Teilchen so gestaltet sind, dass sie ein spektrales Reflexionsvermögen im sichtbaren Spektrum von mindestens 70 % aufweisen.

10. Zusammensetzung nach einem der vorhergehenden Ansprüche, **dadurch gekennzeichnet, dass** die reflektierenden Teilchen eine Dimension von höchstens gleich 250 µm,b vorzugsweise unter 150 und besser 100 µm aufweisen.

11. Zusammensetzung nach einem der vorhergehenden Ansprüche, **dadurch gekennzeichnet, dass** die reflektierenden Teilchen eine Dimension von mindestens gleich 10 µm aufweisen.

12. Zusammensetzung nach einem der vorhergehenden Ansprüche, **dadurch gekennzeichnet, dass** die reflektierenden Teilchen eine Dimension von 20 bis 50 µm aufweisen.

13. Zusammensetzung nach einem der vorhergehenden Ansprüche, **dadurch gekennzeichnet, dass** die reflektierenden Teilchen in der Zusammensetzung in einem Gehalt von 0,1 % bis 20 %, insbesondere von 1 bis 15 %, speziell von 1 bis 10 %, bezogen auf das Gesamtgewicht der Zusammensetzung vorhanden sind.

14. Zusammensetzung nach einem der vorhergehenden Ansprüche, **dadurch gekennzeichnet, dass** die reflektierenden Teilchen die Form von Plättchen oder Kugeln aufweisen.

15. Zusammensetzung nach einem der vorhergehenden Ansprüche, **dadurch gekennzeichnet, dass** das goniochromatische Farbmittel aus Flüssigkristallfarbmitteln und aus mehrschichtigen Interferenzsümkturen gewählt ist.

16. Zusammensetzung nach einem der vorhergehenden Ansprüche, **dadurch gekennzeichnet, dass** das goniochromatische Farbmittel eine mehrschichtige Interferenzstruktur umfasst, die aus den folgenden Strukturen ausgewählt ist: Al/SiO₂/Al/SiO₂/Al; Cr/MgF₂/Al/MgF₂/Cr; MoS₂/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃, MoS₂/SiO₂/Mica-Oxid/SiO₂/MoS₂, Fe₂O₃/SiO₂/Mica-Oxid/SiO₂/Fe₂O₃; TiO₂/SiO₂/TiO_{2;} TiO₂/Al₂O₃/TiO₂; SnO/TiO₂/SiO₂/TiO₂/SnO; Fe₂O₃/SiO₂/Fe₂O₃ und SnO/Mica/TiO₂/SiO₂/TiO₂/Mica/SnO.

17. Zusammensetzung nach Anspruch 16, **dadurch gekennzeichnet, dass** das goniochromatische Farbmittel eine mehrschichtige Interferenzstruktur umfasst, die aus den folgenden Strukturen ausgewählt ist: MoS₂/SiO₂/Al/SiO₂/MoS₂; Fc₂O₃/SiO₂/Al/SiO₂/Fe₂O₃; Fe₂O₃/SiO₂/Fe₂O₃/SiO₂/Fe₂O₃; SnO/TiO₂/SiO₂/TiO₂/SnO; Fe₂O₃/SiO₂/Fe₂O₃ und SnO/Mica/TiO₂/SiO₂/TiO₂/Mica/SnO.

18. Zusammensetzung nach einem der vorhergehenden Ansprüche, **dadurch gekennzeichnet, dass** das goniochromatische Farbmittel in einer Menge von 0,1 % bis 20 %, insbesondere von 2 bis 10 %, bezogen auf das Gesamtgewicht der Zusammensetzung vorhanden sind.

19. Zusammensetzung nach einem der vorhergehenden Ansprüche, **dadurch gekennzeichnet, dass** sie eine Glanzgrundlage von einem durchschnittlichen Glanzwert von größer als 20 umfasst.

20. Zusarmnensetzung nach Anspruch 19, **dadurch gekennzeichnet, dass** sie eine Glanzgrundlage mit einem durchschnittlichen Glanzwert von größer als 50 umfasst.

21. Zusammensetzung nach Anspruch 20, **dadurch gekennzeichnet, dass** sie eine Glanzgrundlage mit einem durchschnittlichen Glanzwert von größer als 70 umfasst.

22. Zusammensetzung nach einem der vorhergehenden Ansprüche, **dadurch gekennzeichnet, dass** sie weiterhin mindestens ein nicht-goniochromatisches Farbmittel umfasst.

23. Zusammensetzung nach Anspruch 22, **dadurch gekennzeichnet, dass** das goniochromatische Farbmittel aus Farbmitteln, monochromen Pigmenten und Perlmutt ausgewählt ist.

24. Zusammensetzung nach einem der vorhergehenden Ansprüche, **dadurch gekennzeichnet, dass** sie goniochromatisrhe Fasern umfasst.

25. Zusammensetzung nach einem der vorhergehenden Ansprüche, **dadurch gekennzeichnet, dass** sie in den folgenden Formel dargereicht wird: einer wasserfreien Form, einer Form von einer öligen oder wässrigen Lösung, einem öligen oder wässrigen Gel, einer Öl-in-Wasser- oder einer Wasser-in-Öl-Emulsion, einer Mehrfachemulsion, einer Öl-in-Wasser-Dispersion auf Grund von Vesikeln, die an der Öl/Wasser-Grenzfläche vorhanden sind.

26. Zusammensetzung nach einem der vorhergehenden Ansprüche, **dadurch gekennzeichnet, dass** sie in der Farm eines flüssigen Glosses vorkommt.

27. Produkt zum Schminken der Lippen umfassend eine Zusammensetzung nach einem der vorhergehenden Ansprüche.

28. Nagellack umfassend eine Zusammensetzung nach einem der Ansprüche 1 bis 25.

29. Teintgrundierung umfassend eine Zusammensetzung nach einem der Ansprüche 1 bis 25.

30. Maskara umfassend eine Zusammensetzung nach einem der Ansprüche 1 bis 25.

31. Verwendung zum Schminken der Haut, der Lippen oder der Phaneren von einer Zusammensetzung nach einem der Ansprüche 1 bis 30.

32. Verfahren zum Schminken von einem Träger, ausgewählt aus Haut, Lippen und Phaneren, umfassend das Aufbringen hintereinander auf den Träger von mindestens einem goniochromatischen Farbmittel und reflektierenden Teilchen, die dazu geeignet sind, auf den Träger mit dem bloßen Auge sichtbare Lichtpunkte zu erzeugen, wobei die reflektierenden Teilchen Teilchen aus Glas sind, das mindestens zum Teil mit mindestens einer Schicht von mindestens einer Metallverbindung und insbesondere von einem Metalloxid überzogen ist.

33. Kit zum Schminken von einem Träger, ausgewählt aus Haut, Lippen und Phaneren, zur Durchfürung des Verfahrens nach Anspruch 32, umfassend eine erste und eine zweite kosmetische Zusammensetzung, die voneinander verschieden sind, wobei die erste Zusammensetzung mindestens reflektierende Teilchen umfasst, wobei die reflektierenden Teilchen Teilchen aus Glas sind, das mindestens zum Teil mit mindestens einer Schicht von mindestens einer Metallverbindung und insbesondere von einem Metalloxid überzogen ist, wobei die zweite Zusammensetzung, einem physiologisch verträglichen Medium, mindestens ein goniochromatisches Farbmittel umfasst, wobei die erste und die zweite Zusammensetzung getrennt konditioniert sind.
